# EUROPEAN PATENT SPECIFICATION

(11) **EP 4 065 123 B1**
(45) Date of publication and mention of the grant of the patent: **03.01.2024**
(21) Application number: 20812042.8
(22) Date of filing: 27.11.2020
(51) Int. Cl.: A61K 31/517, A61P 1/10, A61P 25/36, A61K 31/485

(54) **USE OF 2-PHENYL-6-(1H-IMIDAZOL-1-YL) QUINAZOLINE FOR THE PREVENTION OF ABUSE AND OF SIDE EFFECTS OF AT LEAST ONE OPIOID**
VERWENDUNG VON 2-PHENYL-6-(1H-IMIDAZOL-1-YL)-CHINAZOLIN ZUR VORBEUGUNG DES MISSBRAUCHS UND DER NEBENWIRKUNGEN VON MINDESTENS EINEM OPIOID
UTILISATION DE 2-PHÉNYL-6-(1H-IMIDAZOL-1-YL)QUINAZOLINE POUR LA PRÉVENTION DE L'ABUS ET DES EFFETS SECONDAIRES D'AU MOINS UN OPIOÏDE

(30) Priority: 29.11.2019 WO PCT/EP2019/083041
(43) Date of publication of application: 05.10.2022
(73) Proprietor: Rottapharm Biotech S.r.l., 20900 Monza (IT)
(72) Inventor: ROVATI, Lucio Claudio, 20900 MONZA (IT)
(74) Representative: Cattaneo, Elisabetta
(86) International application number: PCT/EP2020/083713
(87) International publication number: WO 2021/105414

(56) References cited:
- WO-A1-2009/152868
- DAVID A THORN ET AL: "Effects of the imidazoline I 2 receptor agonist 2-BFI on the development of tolerance to and behavioural/physical dependence on morphine in rats : I 2 receptor ligands attenuate morphine tolerance", BRITISH JOURNAL OF PHARMACOLOGY, vol. 173, no. 8, 18 January 2016 (2016-01-18), pages 1363-1372, XP055525182, UK ISSN: 0007-1188, DOI: 10.1111/bph.13435
- MARCO LANZA ET AL: "Analgesic efficacy of CR4056, a novel imidazoline-2 receptor ligand, in rat models of inflammatory and neuropathic pain", JOURNAL OF PAIN RESEARCH, 18 April 2011 (2011-04-18), page 111, XP055525179, GB ISSN: 1178-7090, DOI: 10.2147/JPR.S18353

## Description

### FIELD OF THE INVENTION

The present invention relates to the 2-phenyl-6-(1H-imidazol-1-yl) quinazoline compound, indicated briefly as CR4056, for use in the prevention of opioid-induced adverse events, namely chronic constipation and sedation.

Preferably, the prevention of opioid-induced adverse events occurs during a pharmacological therapy for pain of any aetiology and severity with an opioid, whereby preferably the use consists therefore in the co-administration of a dose, in this sense active, of a 2-phenyl-6-(1H-imidazol-1-yl) quinazoline (CR4056) compound, or of a pharmaceutically acceptable salt thereof, in combination with at least one opioid used at doses that are sub-analgesic per se. More preferably, the present invention also refers to a composition comprising the 2-phenyl-6-(1H-imidazol-1-yl) quinazoline (CR4056) compound and at least one opioid for use in the prevention of opioid-induced adverse events in a non-addicted subject.

### TECHNICAL FIELD

Opioids are the most effective medications used for the management of pain, since they are potent analgesics. However, their use is hindered by several drawbacks that may have serious consequences.

The prototype opioid analgesics, such as morphine (but also e.g. codeine, dextromethorphan, dextropropoxyphene, fentanyl, hydrocodone, hydromorphone, levorphanol, meperidine, methadone, oxycodone, oxymorphone, and their derivatives and/or pharmaceutically acceptable salts), exert their pharmacological effects by engaging the endogenous opioid system, where they act as agonists at the mu-opioid receptor (MOR). The agonist action at MOR in specific brain areas is the responsible for both analgesia and the rewarding effects of opioids (Volkow ND. JAMA Psychiatry 2019;76:208-16).

In addition and unfortunately, MORs are also located in other areas of the central and peripheral nervous system and interaction with their agonists induces adverse events. These may include gastrointestinal effects such as dry mouth, nausea, and especially constipation that may be severe and disabling, or central effects such as dizziness, drowsiness, somnolence, or sedation. Even more importantly, MORs are also located in brainstem areas that regulate breathing, where MOR agonists inhibit neuronal firing: this results in respiratory depression, which is the main cause of death from opioid overdose (Volkow ND. JAMA Psychiatry 2019;76:208-16).

The analgesic and rewarding effects of opioids, but not all of the adverse effects above, are subject to tolerance: thus, patients require increasingly higher doses to obtain the same analgesic (or rewarding) effects, thereby increasing the risk of respiratory depression and fatal overdose.

The rewarding effects of opioids (such as e.g. euphoria), may cause opioid use disorders (OUDs), namely their misuse or abuse, that may trigger addiction and physical dependence.

For the purposes of contextualizing the present invention, it is important to understand the nature and general definition of misuse, abuse and related events (MAREs).

They have been classified by a public-private partnership with the United States Food and Drug Administration (FDA) and their published definitions are the following (Smith SM et al. Pain 2013;154:2287-96):
- *Misuse* refers to the intentional therapeutic use of a drug in an inappropriate way, i.e. other than as prescribed. While there may be similarities to certain definitions of "abuse", the term "misuse" excludes that of "abuse" when the term is restricted to "therapeutic" use;
- *Abuse* consists of any intentional, non-therapeutic use of a drug or substance, even once, for the purposes of achieving a desirable psychological or physiological effect. Opioids are misused or abused for their analgesic effects or rewarding properties. The latter reflects their ability to increase the activity of dopamine neurons in the ventral tegmental area and to increase dopamine release in the nucleus accumbens (Volkow ND. JAMA Psychiatry 2019;76:208-16). Misuse and especially abuse should be distinguished by other related events (MAREs), such as:
- *Addiction,* which is primarily defined as compulsive substance use that occurs despite personal harm or negative consequences (Smith SM et al. Pain 2013;154:2287-96). Addiction involves molecular processes associated with learning, which consolidate automatic behaviours in response to the stimuli associated with the drug (Volkow ND. JAMA Psychiatry 2019; 76:208-16).
- *Dependence* usually identifies *physical* dependence and consists of the manifestations of withdrawal symptoms due to rapid reduction in exposure to the drug when it is discontinued (or when the subject is exposed to an antagonist, e.g. naloxone in the case of opioids) (Smith SM et al. Pain 2013;154:2287-96).

It is essential to underline that physical dependence on opioids is distinct from addiction since they have a different definition and indeed reflect different neuroadaptation processes, but they are often confused in the common language (Volkow ND. JAMA Psychiatry 2019;76:208-16). In addition, while addiction occurs in a smaller subset of opioid users, all patients treated with opioids will develop physical dependence, regardless of abuse.

It is also important to note that some definitions of physical dependence include the development of tolerance (Smith SM et al. Pain 2013;154:2287-96) as they probably reflect similar mechanisms.

Thus, from the foregoing it is essential to indicate that, while abuse depends on opioid rewarding properties due to increased dopaminergic activity, addiction or dependence (and possibly tolerance) are due to neuroadaptive processes: all these phenomena should be therefore clearly distinguished between them.

Therefore, in summary, opioid abuse is one of the leading causes of increased use of opioids, which in turn triggers and favours addiction and accelerates the development of tolerance and dependence, thus leading to the use of higher doses to obtain the same rewarding effects and avoid withdrawal symptoms. Opioid abuse is therefore the ultimate responsible for the use of high doses of opioids, which increase the risk of respiratory depression and fatal overdose.

Indeed, the so-called "opioid crisis" is currently one of the most urgent medical emergencies in the USA and worldwide (NIH. Opioid overdose crisis. https://www.drugabuse.gov/drugs-abuse/opioids/opioid-overdose-crisis#five.

Revised March 2018; Volkow ND and Collins FS. NEJM 2017,377:391-94):
- Every day more than 115 people in the USA die from opioid overdose (mainly respiratory depression)
- Misuse/abuse of opioid analgesics play a major role
- In 2015 an estimated 2 million people in the USA suffered from substance use disorders related to prescription opioids
- 21% to 29% patients prescribed opioids for chronic pain misuse/abuse them
- About 80% heroin users, first misused/abused prescription opioids.

This represents a real "social scourge", as well as a medical problem, whose impact is devastating in the Western world (Manchikanti, Pain Physician. 2017; 20(Suppl. 2):S3-S92). Therefore, the National Institutes of Health (NIH) launched an aggressive effort to develop, among other measures, safe and effective strategies devoid of abuse liability, to manage chronic pain. The US FDA has acknowledged this necessity and, for example, produced a guidance for industry for the development of so-called "abuse-deterrent opioid formulations" (U.S. Department of Health and Human Services, Food and Drug Administration, Center for Drug Evaluation and Research (CDER). Abuse-Deterrent Opioids: Evaluation and Labeling - Guidance for Industry. April 2015). Indeed, when abused for their rewarding effects, prescription opioids even from oral formulations are frequently snorted or injected, which leads to faster uptake in the brain, enhancing their rewarding effects. A few abuse-deterrent formulations have been approved lately, but this does not yet solve the problem of avoiding opioid abuse. Conversely and for the first time, the present invention aims at providing a method which is capable of avoiding and thus of preventing opioid abuse in opioid non-addicted subjects. The 2-phenyl-6-(1H-imidazol-1-yl) quinazoline (CR4056) compound is known as a powerful imidazoline I2 receptor ligand endowed with analgesic properties. The analgesic activity of CR4056 per se has been described in numerous publications utilizing different experimental models.

Previous art in the scientific literature describes that imidazoline I2 receptor agonists potentiate morphine-induced analgesia. Giordani et al. (WO2009/152868) have in fact demonstrated, in a rat experimental model of chronic inflammation induced by a subcutaneous injection in the paw of complete Freund adjuvant (CFA), that CR4056 enhanced synergistically the effect of a morphine low and sub-analgesic dose. This enhancing effect of opioid-induced analgesia is known for other I-2 ligands (Li et al., European Journal of Pharmacology, 669(2011) 59-65) and it has been confirmed for CR4056 also in other studies, e.g. as published by Lanza et al. (British Journal of Pharmacology, 2014: 171 3693-3701) in a post-operative pain model in rats.

I2 receptor agonists also prevent the development of tolerance to the analgesic effects of opioids (Boronat MA et al. Br J Pharmacol 1998,125:175-85; Caprioli G et al. Eur J Pharmacol 2015,769:219-24). More extensively, David A Thorn et al (British Journal of Pharmacology, vol. 173, no. 8, (2016-01-18), pages 1363-1372) report the use of imidazolidine I2 receptor agonists on the development of tolerance to and behavioural/physical dependence on morphine in a combination used for the treatment of pain. Such a combination is described as capable to attenuate the development of tolerance to and behavioural/physical dependence on morphine.

These effects described in previous art should not be confused with the prevention of opioid use disorders such as abuse which is the primary aim that the present invention intends to solve, since they represent completely different phenomena and mechanisms, as described in Smith SM et al. Pain 2013;154:2287-96, and as summarized above. Indeed, tolerance and physical dependence probably derive from similar biological mechanisms and actually the definition of dependence may include the development of tolerance, beside the typical withdrawal symptoms on decrease of opioid blood levels or administration of an antagonist (Smith SM et al. Pain 2013;154:2287-96). With respect to the effects of imidazoline I2 agonists, the prevention of tolerance is probably still related to opioid potentiation, i.e. a potentiated dose may be effective for longer periods of repeated administrations before the onset of tolerance, and the same mechanism may apply to the mitigation of withdrawal effects (i.e. a potentiated dose may be less sensitive to the decrease in blood levels or administration of an antagonist).

As a matter of fact, Giordani et al (WO2009/152868) claimed the use of CR4056 to enhance opioid-induced analgesia and to treat physical dependence, i.e. the withdrawal symptoms from the administration of different substances (including opioids). In addition, they claimed the use of CR4056 as an antidepressant to avoid, among other medical uses as antidepressant, to fall back into episodes of abuse in previously addicted subjects.

### SUMMARY

The present invention is defined in the appended claims. Embodiments not falling within the scope of the appended claims do not form part of the invention. Any references in the description to methods of treatment refer to the compounds, pharmaceutical compositions and medicaments of the present invention for use in a method for treatment of the human (or animal) body by therapy.

The inventors have evaluated the compound CR4056 in experimental animal models that are predictive of drug abuse in humans and more in particular of morphine or other opioids abuse, and they have discovered that CR4056 was capable to prevent opioid abuse in an opioid non -addicted subject. In addition, they have found that CR4056 was also capable to prevent opioid side effects, such as constipation and sedation.

It is worthy to point out that prior art uses are completely different than the use to prevent opioid-abuse in non-addicted subjects which is described in the present invention: the latter is counterintuitive and unexpected, compared with the uses claimed in WO2009/152868. Indeed, Giordani et al (WO2009/152868) even describe something which is opposite to what is described in the present invention, when they claim the use of CR4056 in the enhancement of the opioid pharmacological action in general: in fact, the present invention demonstrates that while opioid analgesia can be potentiated as reported in previous art, abuse liability is not potentiated and, conversely, can be prevented. This effect is surprising: since the mechanisms of opioid analgesia and opioid rewarding effects are the same, as described above (Volkow ND. JAMA Psychiatry 2019;76:208-16), it would be expected that a compound that potentiates morphine-induced analgesia also potentiates morphine rewarding effects, thus favouring abuse, or not limiting it in case low doses of opioid are used but are potentiated to achieve full analgesic and thus possibly full rewarding effects.

Surprisingly, the present invention thus shows how the administration of an opioid dose, thanks to the combination with CR4056, is able to remain free from phenomena of abuse on a group of subjects considered not-opioid-dependent, non-addicted patients.

A fortiori, the invention is even more surprising when considering the opioid side effects, specifically constipation and sedation.

In fact, the present invention demonstrates that the combination of at least one opioid and the compound CR4056 allows to potentiate opioid analgesia, however it does not increase its side effects, specifically constipation and sedation. Therefore, from a medical point of view the present disclosure relates to the prevention of opioid abuse in a non-addicted subject who receives opioids for the treatment of pain. This objective is achieved by the administration of the lowest possible dose of an opioid which is able to exert maximal analgesia thanks to potentiation by CR4056, but which is devoid of abuse liability and at the same time of typical opioid adverse effects such as constipation and sedation, since none of the latter is potentiated by CR4056 as demonstrated here for the first time.

The inventors have therefore surprisingly found a method to prevent the abuse of opioid drugs in an opioid non-addicted subject and some opioid adverse effects, notwithstanding maximal analgesia and thus to be used preferably in the course of pain therapy.

In a first aspect, the invention therefore concerns a compound of formula 2-phenyl-6-(1H-imidazol-1-yl) quinazoline (CR4056) or a pharmaceutically acceptable salt thereof for use in the prevention of side effects to at least one opioid, wherein the side effects are selected from chronic constipation and sedation.

In the present invention when the term is indicated
- "opioid" means a substance capable of acting as agonist at the mu-opioid receptors (MORs) and include e.g. morphine, codeine, dextromethorphan, dextropropoxyphene, fentanyl, hydrocodone, hydromorphone, levorphanol, meperidine, methadone, oxycodone, oxymorphone, and their derivatives and/or pharmaceutically acceptable salts. Opioids are also generally referred to as opioid drugs, or opiates;
- "abuse" means any non-therapeutic and intentional use of a drug or substance, in this case an opioid drug, to achieve a desirable physiological or psychological effect;
- "non-addicted subject" means a subject who is not addicted by use of an opioid.

As above indicated, it is known in the art that imidazoline-2 (12) agonists are capable to potentiate opioid-induced analgesia, thus making sub-analgesic doses of an opioid fully analgesic. Therefore it was logically expected that other unwanted effects of opioids would also be potentiated, such as the rewarding effects leading to opioid abuse and the development of side effects such as opioid-induced constipation and/or sedation. Contrarily to what was expected the inventors surprisingly showed that, while enhancing the analgesic effects of low doses of opioid, the administration of 2-phenyl-6-(1H-imidazol-1-yl) quinazoline (CR4056) did not lead to opioid abuse nor to the development of constipation or sedation, thus preventing them.

Preferably, the prevention of abuse to an opioid consists in the co-administration of a dose, in this respect active, of CR4056 and a sub-analgesic dose of an opioid in an opioid non-addicted subject.

According to another aspect, the invention therefore concerns a composition comprising a compound of formula 2-phenyl-6-(1H-imidazol-1-yl) quinazoline (CR4056) or a pharmaceutically acceptable salt thereof and at least one opioid for use in the prevention of side effects to the opioid, wherein the side effects are chronic constipation or sedation.

According to the present disclosure, CR4056 is therefore able of enhancing the analgesic effect of the sub-analgesic dose of the opioid drug, as known in the art, but preventing the development of abuse because this latter effect is not enhanced and remains absent with the dosage of the used opioid drug.

The prevention of abuse of an opioid according to a preferred embodiment occurs during a pharmacological pain therapy.

In a further aspect, the present invention relates to the aforementioned co-administration or pharmaceutical combination for the prevention of the occurrence of side effects typical of opioids and which are particularly debilitating, especially in elderly patients, such as opioid-induced constipation (OIC) and opioid-induced sedation. The latter side effect is particularly relevant, since opioid-induced sedation precedes and often predicts the dangerous side effect consisting in respiratory depression, which is the most serious and life-threating adverse event of therapy with opioids (Pasero K. Journal of PeriAnesthesia Nursing, 2009, 24:186-90; Montandon G. et al. Anesthesiology 2016; 125:889-903).

As a matter of fact and according to the present invention, the pharmaceutical combination can correspond to either a composition comprising as active substances CR4056, or a pharmaceutically acceptable salt thereof, and at least one opioid, or a kit for the simultaneous, sequential or separate administration of the two active ingredients as analgesic treatment in the prevention of opioid side effects which are OIC or sedation

In a further aspect, therefore, the invention relates to a pharmaceutical kit for the chronic therapeutic treatment of pain, comprising:
- one or more discrete units of a compound of formula 2-phenyl-6-(1H-imidazol-1-yl) quinazoline (CR4056) or a pharmaceutically acceptable salt thereof and
- one or more discrete units of an opioid drug
for simultaneous, sequential or separate use in the prevention the side effects of the opioid drug, wherein the side effects are selected from chronic constipation and sedation.

### BRIEF DESCRIPTION OF THE FIGURES

Figure 1 shows the effects of different doses of morphine (panel A), CR4056 (B), and their combination (C) on inflammatory pain induced by complete Freund adjuvant (CFA) (*P<0.05 and **P<0.01 vs control; statistical analysis of sham animals not performed);
Figure 2 shows the effect of CR4056 on defecation in normal rats, in comparison to codeine alone and to the CR4056-codeine combination treatment (Data represent the mean faecal number ± SEM (n=6 per group) evaluated 16 h after drug treatment);
Figure 3 shows the effect of CR4056 on morphine-induced constipation in rats (The faecal number was evaluated 16 h after drug treatment. Panel A: Morphine (10 mg/kg) induced a significant decrease in faecal number (*P<0.05 vs vehicle). The co-treatment of morphine with an analgesic dose of CR4056 did not further reduce stool number in comparison with morphine alone. Panel B: morphine and CR4056 co-administered at non-analgesic doses per se (1 mg/kg) did not induce constipation in rats, although this combination exerts a synergistic full analgesic effect as described above);
Figure 4 shows the dose-response sedative effect of morphine in the rodent open field test (panel A) and absence of sedation with low dose morphine combined with low dose CR4056 (panel B);
Figure 5 shows the absence of sedation or of potentiation of sedation in the rodent open field test with different doses of morphine combined with low or full analgesic doses of CR4056;
Figure 6 shows the effects of a sedative dose of morphine and its combination with CR4056 on sedation scores (panel A) and rotarod test (panel B);
Figure 7 shows the effects of a low, non-sedative dose of morphine with or without CR4056 low or full analgesic doses on sedation scores (panel A) and rotarod test (panel B).

The features and advantages of the present invention will be evident from the following detailed description and from the embodiments provided by way of illustrative examples.

### DETAILED DESCRIPTION OF THE INVENTION

The invention therefore relates to a compound of formula 2-phenyl-6-(1H-imidazol-1-yl) quinazoline (CR4056) or a pharmaceutically acceptable salt thereof for use in the prevention of side effects to at least one opioid, selected from chronic constipation and a sedative effect where the latter precedes and may predict the most dangerous opioid side effect consisting in respiratory depression.

The prevention of the abuse occurs during a pharmacological pain therapy and consists in the co-administration of 2-phenyl-6-(1H-imidazol-1-yl) quinazoline (CR4056) or a pharmaceutically acceptable salt thereof with the at least one opioid in an opioid non-addicted subject.

Preferably, the at least one opioid is a mu-opioid receptor (MOR) agonist selected from the group consisting of morphine, codeine, dextromethorphan, dextropropoxyphene, fentanyl, hydrocodone, hydromorphone, levorphanol, meperidine methadone, oxycodone, oxymorphone, and/or their pharmaceutically acceptable salts. More preferably, the at least one opioid is morphine or a pharmaceutically acceptable salt thereof.

During the pharmacological therapy of pain, the compound of formula 2-phenyl-6-(1H-imidazol-1-yl) quinazoline (CR4056) or a pharmaceutically acceptable salt thereof and/or the at least one opioid are preferably administered by the oral or any topical or parenteral routes of administration, including e.g. the transdermal, subcutaneous, intramuscular, intracerebroventricular, intrathecal, intranasal, inhaled, rectal routes.

During the pharmacological therapy of pain, the at least one opioid is in an amount which is suboptimal as analgesic per se, but able to be potentiated while devoid of abuse and/or some side effect liability.

In fact, the at least one opioid should be in an amount able to be potentiated to provide full or almost full analgesia, but devoid or almost devoid of abuse and some side effect liability. The weight ratio between the compound of formula 2-phenyl-6-(1H-imidazol-1-yl) quinazoline (CR4056) and the at least one opioid is depending on the analgesic potency, side effect profile and route of administration of the opioid: according to the present invention, it is deduced that it is generally possible to reduce the daily therapeutic dosage of an at least one opioid from 1 to over 5 folds compared with the use as monotherapy.

Preferably, the at least one opioid in an amount which is suboptimal per se is morphine or one of its pharmaceutically acceptable salts.

The compound of formula 2-phenyl-6-(1H-imidazol-1-yl) quinazoline (CR4056) or a pharmaceutically acceptable salt thereof is orally administered in an amount in the range from 10 to 400 mg/die.

In another aspect the invention relates to a composition comprising a compound of formula 2-phenyl-6-(1H-imidazol-1-yl) quinazoline (CR4056) or a pharmaceutically acceptable salt thereof and at least one opioid for use in the prevention of some side effects to that opioid, which are chronic constipation or a sedative effect. Preferably the composition comprises the at least one opioid in an amount which is suboptimal as analgesic per se, but able to be potentiated while devoid of abuse and/or some side effect liability, and the compound of formula 2-phenyl-6-(1H-imidazol-1-yl) quinazoline (CR4056) or a pharmaceutically acceptable salt thereof in an amount in the range from 10 to 400 mg/die.

In the composition according to the invention, the at least one opioid is a MOR agonist preferably selected from the group consisting of morphine, codeine, dextromethorphan, dextropropoxyphene, fentanyl, hydrocodone, hydromorphone, levorphanol, meperidine methadone, oxycodone, oxymorphone, and/or their pharmaceutically acceptable salts. More preferably, the at least one opioid is morphine or a pharmaceutically acceptable salt thereof.

The composition for the use can be formulated through a pharmaceutical form suitable for the desired administration. The composition for the use can therefore also comprise pharmaceutically acceptable excipients and technologies suitable for the final route of administration.

The pharmaceutical compositions for the use according to the invention can hence be prepared by adding to the compound CR4056, or a pharmaceutically acceptable salt thereof, and to the at least one opioid as selected, pharmacologically acceptable inactive ingredients such as excipients, binders, dispersants, colorants, humectants, commonly used for the preparation of tablets, capsules, pills, solutions, suspensions, emulsions in the case of oral administration. Isotonic solutions and other media are also contemplated which can be locally or parenterally administered, (including e.g. the transdermal, subcutaneous, intramuscular, intracerebroventricular, intrathecal, intranasal, inhaled, rectal routes).

All the pharmaceutical compositions for the uses described above can be prepared by methods known in the state of the art in relation to the specific administration route.

In another aspect the invention relates to a pharmaceutical kit comprising:
- one or more discrete units of the compound of formula 2-phenyl-6-(1 Himidazol-1-yl) quinazoline (CR4056) or a pharmaceutically acceptable salt thereof and
- one or more discrete units of an opioid
for simultaneous, sequential or separate use in the prevention of side effects to at least one opioid, selected from chronic constipation and a sedative effect.

Below are provided examples of embodiments of the present invention in an illustrative and fashion.

### EXAMPLES

### Example 1

*CR4056 does not synergize with opioids on abuse* Conditioned place preference (CPP) is a commonly used experimental paradigm that measures reward behaviours associated with drug abuse. This test exploits the tendency of rats to associate the environment where they are located with the effect of drugs administered during a conditioning period. The experiment was done in a wooden apparatus consisting of two lateral chambers (30 x 25 cm, length x width) with different visual and tactile properties and a central "neutral" chamber, smaller than the others (12 x 25 cm, length x width) with total white floor and walls. The lateral chambers had different decoration of walls (points or stripes) and different type of floor (smooth or rough). These differences can be recognized by rats, and thus associated with the effect of drug or vehicle treatment. All walls were 38.5 cm high. Four days before the preconditioning phase, the light/dark cycle was inverted; all phases of experiments were performed during the dark phase. On the preconditioning day (day 1), Wistar rats (Charles River) were placed in the neutral chamber and for 15 min they were free to explore all the environments of the apparatus (van der Kam E.L. et al. Pain, 2008, 136:373-379). The time spent in each chamber was recorded with a video tracking software (Ethovision XT 13, Noldus information technology). Rats that spent over 60% of time in one of two lateral chambers were not admitted to the conditioning phase: for admitted animals, the "preferred" chamber was therefore the one where a rat spent between 50% and 60% of time during preconditioning (while the "unpreferred" chamber was the opposite one). After preconditioning, rats were randomly assigned to the experimental groups (n=16). A total of 256 rats were used in this model. During the 2 days of the conditioning phase rats were treated with the test drug(s) (day 2) or vehicle (day 3). On the first conditioning day rats were treated with the test drug(s) and were confined in the preconditioning unpreferred lateral chamber (now defined paired chamber), with no access to the other chambers of the apparatus for 40 min. On the second day of conditioning rats were treated with vehicle and were located in the opposite chamber (unpaired chamber). The control group received vehicle in both chambers. CR4056 or its vehicle were administered 40 min before conditioning, whereas morphine or its vehicle were administered immediately before conditioning. There was no treatment on the test day (day 4), when rats were placed in the neutral chamber with the possibility to explore all chambers of the apparatus. The time spent in each chamber was recorded. A significant difference between the mean time spent in the paired vs the unpaired chamber on the test day suggests the potential for a drug to induce preference (i.e. to induce a rewarding effect and thus to exert an abuse liability) (Mueller D. et al., Behavioural Brain Research 2002, 136:389-397).

In this set of experiments, three doses of morphine were administered alone. Results are reported in Table 1 and show that morphine induced preference for the paired chamber in a dose dependent manner already after acute administration. Actually, 1 mg/kg of morphine did not induce preference while doses of 2.5 and 5 mg/kg induced a statistically significant difference of time spent by rats in the paired vs the unpaired chamber.

**Table 1: "Conditioned Place Preference" (CPP): Effects of morphine, CR4056, and their combination after single administration**

| **Treatment** | **Seconds in Paired chamber (P)** | **Seconds in Neutral chamber (N)** | **Seconds in Unpaired chamber (U)** | **Δ(P-U)±ES** | **%(P-U)** |
|---|---|---|---|---|---|
| Saline | 344 | 197 | 359 | -15.2 ± 9.3 | -2.3 |
| Morphine (1 mg/Kg) | 348 | 211 | 341 | 7.6 ± 9.4 | 1.2 |
| Morphine (2.5 mg/Kg) | 411 | 198 | 291 | 120.5(*)± 20.7 | 17.1 |
| Morphine (5 mg/Kg) | 358 | 276 | 266 | 132.1 (*) ± 26.5 | 20.2 |
| CR4056 (6 mg/kg) + Saline | 326 | 220 | 354 | -5.6 ± 86 | -0.5 |
| CR4056 (6 mg/kg) + Morphine (1 mg/kg) | 340 | 213 | 347 | -7.9 ± 9.9 | -1.1 |
| CR4056 (6 mg/kg) + Morphine (5 mg/kg) | 417 | 194 | 289 | 128.1 ± 16.2(*) | 18.1 |

| | | | | | |
|---|---|---|---|---|---|
| (*): Paired t-test <0.05 | | | | | |

The bottom half of Table 1 reports the effects of CR4056 and of the co-administration of morphine with CR4056. The full analgesic dose of CR4056 (6 mg/kg) did not induce place preference alone and did not potentiate morphine either at the CPP inactive dose of 1 mg/kg or at the CPP active dose of 5 mg/kg morphine. In Table 2 are reported the results of conditioned place preference performed after repeated administrations. CR4056 (1 mg/kg) and morphine (1 mg/kg) alone or in co-administration were administered every other day. After 8 days of conditioning the test day was held as described above.

**Table 2: "Conditioned Place Preference" (CPP): Effects of morphine and its combination with CR4056 after repeated administrations**

| **Treatment** | **Seconds in Paired chamber (P)** | **Seconds in Neutral chamber (N)** | **Seconds in Unpaired chamber (U)** | **Δ(P-U)±ES** | **%(P-U)** |
|---|---|---|---|---|---|
| Saline | 321 | 253 | 326 | -5 ± 12 | -1 |
| Morphine (1mg/kg) | 390 | 215 | 295 | 95 ± 17 (*) | +14 |
| CR4056 (1 mg/kg) + Morphine (1mg/kg) | 331 | 246 | 323 | 8 ± 13 | +1 |

| | | | | | |
|---|---|---|---|---|---|
| (*): Paired t-test <0.05 | | | | | |

The results showed that 1 mg/kg of morphine (inactive after single conditioning) induced preference after repeated conditionings. There was, indeed, a significant preference for the paired chamber in rats treated with morphine 1 mg/kg alone. The co-administration of CR4056 and morphine, as already noted in the experiment with single conditioning, did not induce preference for the paired chamber.

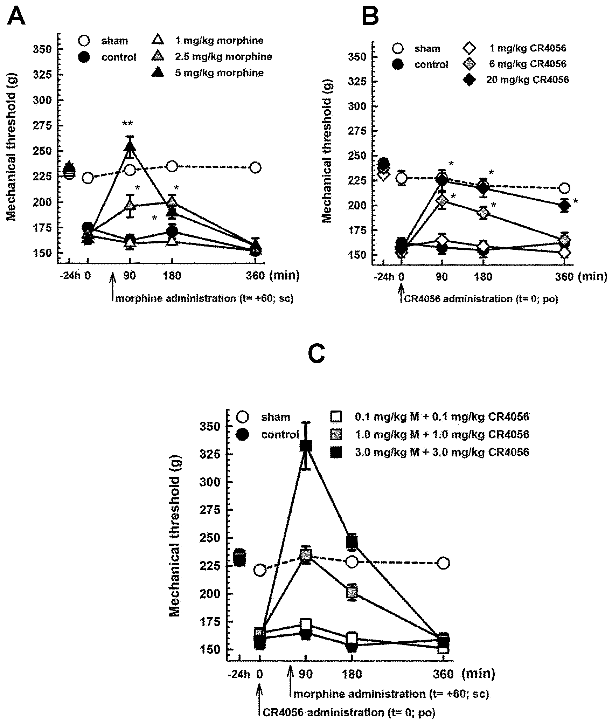

Of note, 1 mg/kg of morphine is not analgesic per se and it is potentiated to full analgesia by the non-analgesic dose of 1 mg/kg CR4056: Figure 1 depicts the known analgesic effects of CR4056, morphine and their combination in the inflammatory pain paradigm induced by CFA.

This set of experiments shows therefore that CR4056 does not potentiate experimental abuse liability of single dose morphine and can even prevent the development of abuse after repeated doses of morphine. This finding is surprisingly at variance with the known effects of CR4056 and other imidazoline-12 agonists in potentiating morphine analgesia and, consequently, in preventing the development of tolerance and of withdrawal symptoms.

### Example 2

### CR4056 does not synergize with opioids on opioid-induced constipation

To rule out the possibility that synergism existed for the typical adverse reactions to opioids, CR4056 was studied in a rodent model of opioid-induced constipation. Activation of mu-opioid receptors (MORs) in the gastrointestinal tract inhibits propulsive peristalsis, thus slowing intestinal transit. Opioid-induced constipation is common and can arise at any time during treatment, because tolerance develops to the analgesic effects and upper gastrointestinal motility effects, but not to colon motility effects (Akbarali H.I. et al. Neurogastroenterol Motil. 2014, 26:1361-1367).

The aim of the present experiment was to evaluate if a possible synergistic activity between CR4056 and morphine or codeine occurred on constipation, a typical adverse effect induced by opioids.

The experiments were conducted according to the procedure described by Harada et al. (J. Pharmacol. Exp. Ther. 2017, 362: 78-84). Wistar rats were used in this model. Fecal excretion was evaluated at the end of the dark phase of the light/dark cycle, when most activities and feeding of albino rats take place. Drugs were administered 2 h before the dark phase of the light/dark cycle, and the feces were collected and counted 16 h after drug treatment. In this experimental paradigm, CR4056 1 or 6 mg/kg was administered orally 15 min before morphine or codeine. A full analgesic dose of morphine (10 mg/kg subcutaneous) or codeine (12 mg/kg oral) were administered 15 minutes after CR4056 or vehicle.

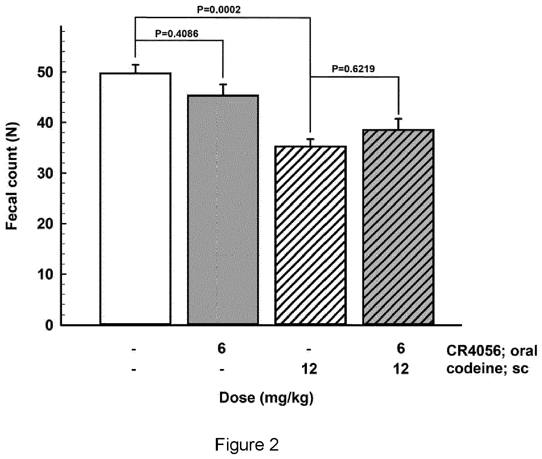

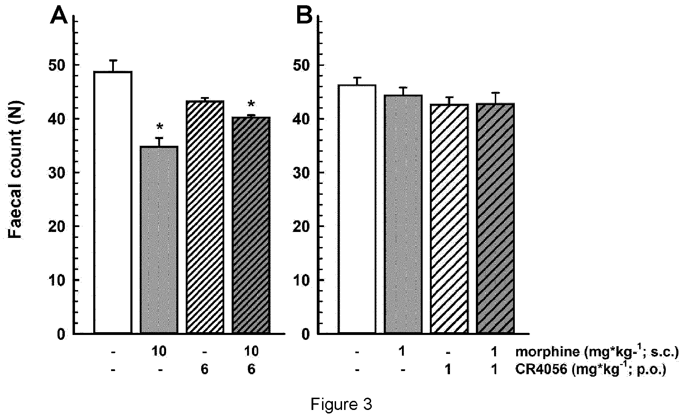

The results obtained are shown in Figures 2 and 3.

Figure 2 shows the effect of the administration of CR4056 6 mg/kg on defecation in comparison to codeine 12 mg/kg and to the CR4056-codeine combination treatment. Codeine alone induced a significant decrease in defecation compared with vehicle or CR4056. The co-administration of a full analgesic dose of CR4056 did not worsen codeine-induced constipation and thus did not show a synergistic or additive modulation of this opioid adverse effect.

Figure 3 Panel A shows the effect on defecation of full analgesic doses of morphine and CR4056, 10 mg/kg or 6 mg/kg, respectively. Morphine significantly reduced faecal count (P<0.05 vs vehicle; Tukey's multiple comparisons test) and thus induced constipation. Under these experimental conditions, co-administration of CR4056 with morphine did not worsen opioid- induced constipation; rather there was a trend for improvement. Importantly, the low doses of CR4056 1 mg/kg and morphine 1 mg/kg that act synergistically in analgesia (see above), did not induce constipation when co-administered (Figure 3, Panel B).

This set of experiments shows therefore that regardless of the dose administered, CR4056 did not worsen morphine or codeine-induced constipation. Most importantly, there was no constipation when the low doses of CR4056 and morphine were combined, despite the known synergistic analgesic effects described above.

### Example 3

### CR4056 does not synergize with opioids on opioid-induced sedation

To confirm that there is no synergism also for other typical adverse reactions to opioids, CR4056 was studied in rodent models of opioid-induced sedation.

The so-called open field test was used as a first model and the experiments were performed according to the procedure described by Haleem and Nawaz (Pain, 2017, 18:19-28). Drugs were administered 60 min (CR4056 or its vehicle, p.o.) and 30 min (morphine or its vehicle, s.c.) before the open field test. The video tracking was performed using the software Ethovision XT 13 (Noldus Information Technology), to assess the "distance travelled" as the primary parameter of sedation in this model. The test was performed every day after 4 days of repeated treatment.

The second model consisted of the rotarod performance test for the evaluation of the impairment of motor coordination as a sedation effect (Chuck T.L. et al. Life Sci, 2006, 79:154-161). The rotarod paradigm consists of a rotating cylinder suspended above the floor, on which rodents try to stay in order to avoid falling to the ground. The test had a duration of 2 days. On day 1 each rat did four trials on the rotarod apparatus (Rota-Rod/RS, Panlab, Spain) at a constant speed of 5 rpm during the first and second trials, and of 10 rpm during the third and fourth trials. For each trial, the time of endurance on rotarod (latency) was recorded and the mean value (2 min.) was used as the cut-off during the selection day. The rats that passed the selection (80%, while 20% had to be discarded), were randomly assigned to the experimental groups on day 2. The apparatus was set to acceleration mode: the speed of rotation increased from 0 to 40 rpm in 5 min that was the cut-off of the experiment. Four trials were performed during the test day: the first one immediately before treatment, and the following trials 15, 30 and 45 min after treatment. Sedation consisted in a worsening of performance.

In addition, sedation was scored before each rotarod testing by the sedation rating scale. This scale consists of a panel of 8 parameters (ocular ptosis, head position, posture, muscle tone, spontaneous movements, exploration, righting reflex, corneal reflex) recorded by the operator after drug administration (Chuck T.L. et al. Life Sci, 2006, 79:154-161). At each time point, a score from 0 (maximum sedation) to 5 (absent sedation) was assigned to each of the 8 parameters. The sum of the scores produced the total score.

Results of these experiments are reported in the figures that follow.

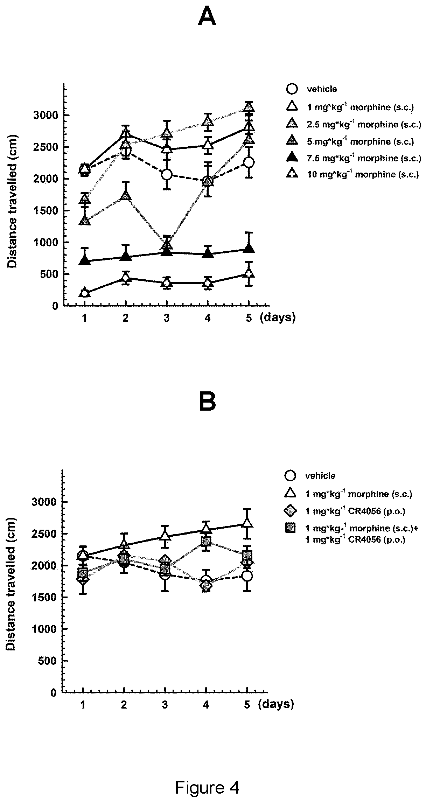

Figure 4A reports the effect of morphine on locomotor activity in the open field test. The results showed a clear sedative effect of morphine at doses higher than 5 mg/kg. Lower doses of morphine were not sedative and, in line with literature data in rodents (Nestler E. J. et al. Semin. Neurosci. 1997, 9: 84-93), there was a modest sensitization in the range 1-2.5 mg/kg, as reflected by increased locomotor activity over the 4 days of repeated treatment.

Notably, a low dose of 1 mg/kg CR4056 which is able to potentiate the analgesic effect of a low dose of morphine 1 mg/kg (see above) is not sedative per se and it is not able to potentiate this morphine low dose to exert a sedative effect (Figure 4B).

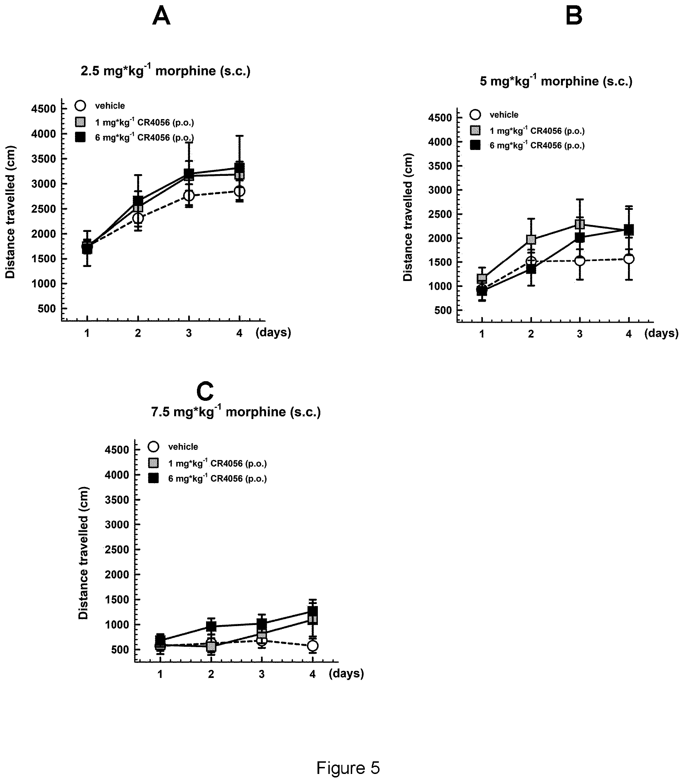

Figure 5 shows that CR4056 did not change the locomotor activity profile of non-sedative and sedative doses of morphine (2.5, 5 and 7.5 mg/kg in panels A, B, or C, respectively) at low or full analgesic doses (1 and 6 mg/kg, respectively).

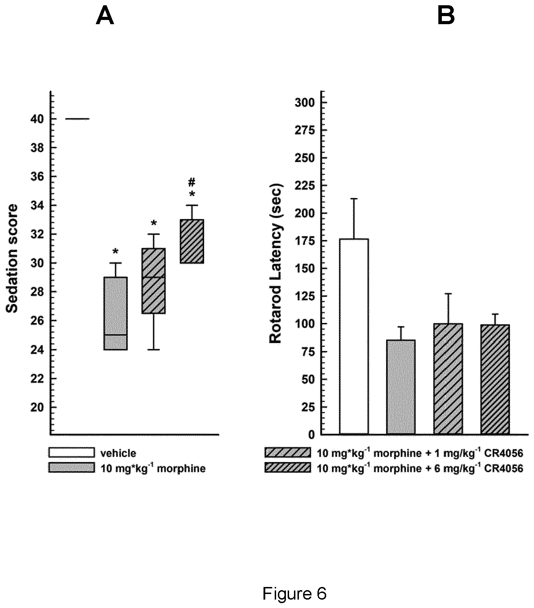

The results obtained from the sedation rating scale and rotarod test are reported in figure 6 (panels A and B). A dose of 10 mg/kg morphine was clearly sedative in these models compared with controls, and was therefore used for the evaluation of possible synergistic interactions with CR4056. Low and high doses of CR4056 (1 and 6 mg/kg, respectively) did not further worsen but even tended to improve the performance of rats treated with 10 mg/kg morphine, with a significant difference in sedation scores at the higher dose of CR4056.

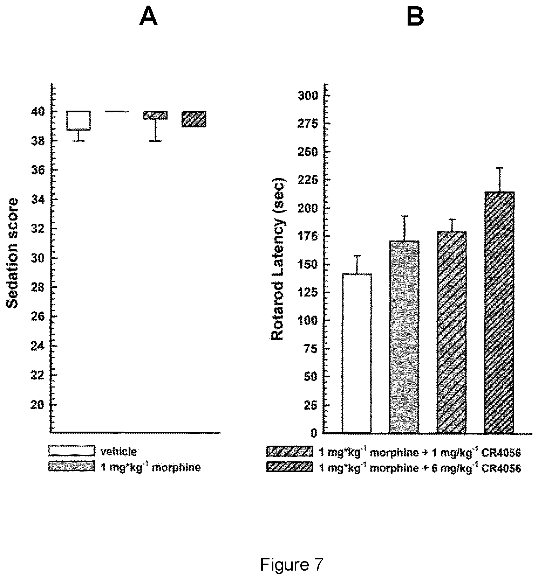

As shown in Figure 7, there were no significant changes in performance vs. control rats when morphine was administered at a non-sedative dose of 1 mg/kg, alone or in combination with different doses of CR4056.

## Claims

1. A compound of formula 2-phenyl-6-(1H-imidazol-1-yl) quinazoline (CR4056) or a pharmaceutically acceptable salt thereof for use in the prevention of side effects to at least one opioid, wherein the side effects are selected from chronic constipation and sedation.

2. The compound for use according to claim 1, wherein the prevention of side effects to at least one opioid occurs during a pharmacological pain therapy and consists in the co-administration of 2-phenyl-6-(1H-imidazol-1-yl) quinazoline (CR4056) or a pharmaceutically acceptable salt thereof with the at least one opioid.

3. The compound for use according to anyone of claims 1-2, wherein the at least one opioid is a mu-opioid receptor (MOR) agonist selected from the group consisting of morphine, codeine, dextromethorphan, dextropropoxyphene, fentanyl, hydrocodone, hydromorphone, levorphanol, meperidine methadone, oxycodone, oxymorphone, and/or their pharmaceutically acceptable salts.

4. The compound for use according to claim 3, wherein the at least one opioid is morphine or a pharmaceutically acceptable salt thereof.

5. The compound for use according to anyone of claims 2-4, wherein the compound of formula 2-phenyl-6-(1H-imidazol-1-yl) quinazoline (CR4056) or a pharmaceutically acceptable salt thereof and/or the at least one opioid are administered by the oral or any topical or parenteral routes of administration.

6. The compound for use according to claims 3 to 5, wherein the compound of formula 2-phenyl-6-(1H-imidazol-1-yl) quinazoline (CR4056) or a pharmaceutically acceptable salt thereof is administered in an amount in the range from 10 to 400 mg/die.

7. A composition comprising a compound of formula 2-phenyl-6-(1H-imidazol-1-yl) quinazoline (CR4056) or a pharmaceutically acceptable salt thereof and at least one opioid for use in the prevention of side effects to that opioid, wherein the side effects are selected chronic constipation and sedation, and wherein the compound of formula 2-phenyl-6-(1H-imidazol-1-yl) quinazoline (CR4056) or a pharmaceutically acceptable salt thereof is administered in an amount in the range from 10 to 400 mg/die.

8. The composition for use according to claim 7, wherein the at least one opioid is a MOR agonist selected from the group consisting of morphine, codeine, dextromethorphan, dextropropoxyphene, fentanyl,hydrocodone, hydromorphone, levorphanol, meperidine methadone, oxycodone, oxymorphone, and/or their pharmaceutically acceptable salts.

9. The composition for use according to claim 7 or 8, wherein the at least one opioid is morphine or a pharmaceutically acceptable salt thereof.

10. The composition for use according to anyone of claims 7-10, wherein the pharmacological composition comprises also pharmaceutically acceptable excipients suitable for the final form of administration.

11. A pharmaceutical kit comprising:
- one or more discrete units of the compound of formula 2-phenyl-6-(1Himidazol-1-yl) quinazoline (CR4056) or a pharmaceutically acceptable salt thereof and
- one or more discrete units of an opioid
for simultaneous, sequential or separate use in the prevention of side effects to at least one opioid, wherein the side effects are selected from chronic constipation and sedation.

## Patentansprüche

1. Verbindung der Formel 2-Phenyl-6-(1H-Imidazol-1-yl)-chinazolin (CR4056) oder ein pharmazeutisch verträgliches Salz davon zur Verwendung bei der Vorbeugung von Nebenwirkungen von mindestens einem Opioid, wobei die Nebenwirkungen aus chronischer Verstopfung und Sedierung ausgewählt sind.

2. Verbindung zur Verwendung nach Anspruch 1, wobei die Vorbeugung von Nebenwirkungen auf mindestens ein Opioid während einer pharmakologischen Schmerztherapie erfolgt und in der gemeinsamen Verabreichung von 2-Phenyl-6-(1H-imidazol-1-yl)-chinazolin (CR4056) oder einem pharmazeutisch akzeptablen Salz davon mit dem mindestens einen Opioid besteht.

3. Verbindung zur Verwendung nach einem der Ansprüche 1 - 2, wobei das mindestens eine Opioid ein Mu-Opioidrezeptor (MOR)-Agonist ist, ausgewählt aus der Gruppe bestehend aus Morphin, Codein, Dextromethorphan, Dextropropoxyphen, Fentanyl, Hydrocodon, Hydromorphon, Levorphanol, Meperidin, Methadon, Oxycodon, Oxymorphon und/oder deren pharmazeutisch akzeptablen Salzen.

4. Verbindung zur Verwendung nach Anspruch 3, wobei das mindestens eine Opioid Morphin oder ein pharmazeutisch akzeptables Salz davon ist.

5. Verbindung zur Verwendung nach einem der Ansprüche 2 - 4, wobei die Verbindung der Formel 2-Phenyl-6-(1H-imidazol-1-yl)-chinazolin (CR4056) oder ein pharmazeutisch annehmbares Salz davon und/oder das mindestens eine Opioid auf oralem oder einem beliebigen topischen oder parenteralen Verabreichungsweg verabreicht werden.

6. Verbindung zur Verwendung nach einem der Ansprüche 3 bis 5, wobei die Verbindung der Formel 2-Phenyl-6-(1H-imidazol-1-yl)-chinazolin (CR4056) oder ein pharmazeutisch verträgliches Salz davon in einer Menge im Bereich von 10 bis 400 mg/Tag verabreicht wird.

7. Zusammensetzung, umfassend eine Verbindung der Formel 2-Phenyl-6-(1H-imidazol-1-yl)-chinazolin (CR4056) oder ein pharmazeutisch annehmbares Salz davon und mindestens ein Opioid zur Verwendung bei der Vorbeugung von Nebenwirkungen dieses Opioids, wobei die Nebenwirkungen ausgewählt sind aus chronischer Verstopfung und Sedierung, und wobei die Verbindung der Formel 2-Phenyl-6-(1H-imidazol-1-yl)-chinazolin (CR4056) oder ein pharmazeutisch akzeptables Salz davon in einer Menge im Bereich von 10 bis 400 mg/Tag verabreicht wird.

8. Zusammensetzung zur Verwendung gemäß Anspruch 7, wobei das mindestens eine Opioid ein MOR-Agonist ist, der aus der Gruppe ausgewählt ist, die aus Morphin, Codein, Dextromethorphan, Dextropropoxyphen, Fentanyl, Hydrocodon, Hydromorphon, Levorphanol, Meperidin, Methadon, Oxycodon, Oxymorphon und/oder ihren pharmazeutisch akzeptablen Salzen besteht.

9. Zusammensetzung zur Verwendung nach Anspruch 7 oder 8, wobei das mindestens eine Opioid Morphin oder ein pharmazeutisch akzeptables Salz davon ist.

10. Zusammensetzung zur Verwendung nach einem der Ansprüche 7-10, wobei die pharmakologische Zusammensetzung auch pharmazeutisch annehmbare Hilfsstoffe umfasst, die für die endgültige Verabreichungsform geeignet sind.

11. Pharmazeutischer Satz, umfassend:
- eine oder mehrere diskrete Einheiten der Verbindung der Formel 2-Phenyl-6-(1Himidazol-1-yl)-chinazolin (CR4056) oder ein pharmazeutisch akzeptables Salz davon und
- eine oder mehrere diskrete Einheiten eines Opioids
zur gleichzeitigen, aufeinanderfolgenden oder getrennten Verwendung bei der Vorbeugung von Nebenwirkungen auf mindestens ein Opioid, wobei die Nebenwirkungen aus chronischer Verstopfung und Sedierung ausgewählt sind.

## Revendications

1. Composé de formule 2-phényl-6-(1H-imidazol-1-yl) quinazoline (CR4056) ou un de ses sels pharmaceutiquement acceptables pour la prévention des effets secondaires d'au moins un opioïde, dans lequel les effets secondaires sont choisis parmi la constipation chronique et la sédation.

2. Composé à utiliser selon la revendication 1, dans lequel la prévention des effets secondaires d'au moins un opioïde se produit au cours d'un traitement pharmacologique de la douleur et consiste en la co-administration de 2-phényl-6-(1H-imidazol-1-yl) quinazoline (CR4056) ou d'un de ses sels pharmaceutiquement acceptables avec l'au moins un opioïde.

3. Composé à utiliser selon l'une quelconque des revendications 1-2, dans lequel l'au moins un opioïde est un agoniste du récepteur mu-opioïde (MOR) choisi dans le groupe constitué par la morphine, la codéine, le dextrométhorphane, le dextropropoxyphène, le fentanyl, l'hydrocodone, l'hydromorphone, le lévorphanol, la mépéridine, la méthadone, l'oxycodone, l'oxymorphone, et/ou leurs sels pharmaceutiquement acceptables.

4. Composé à utiliser selon la revendication 3, dans lequel l'au moins un opioïde est la morphine ou un sel pharmaceutiquement acceptable de celle-ci.

5. Composé à utiliser selon l'une quelconque des revendications 2 à 4, dans lequel le composé de formule 2-phényl-6-(1H-imidazol-1-yl) quinazoline (CR4056) ou un de ses sels pharmaceutiquement acceptables et/ou l'au moins un opioïde sont administrés par voie orale ou par voie topique ou parentérale.

6. Composé à utiliser selon les revendications 3 à 5, dans lequel le composé de formule 2-phényl-6-(1H-imidazol-1-yl) quinazoline (CR4056) ou un de ses sels pharmaceutiquement acceptables est administré dans une quantité comprise entre 10 et 400 mg/die.

7. Composition comprenant un composé de formule 2-phényl-6-(1H-imidazol-1-yl) quinazoline (CR4056) ou un sel pharmaceutiquement acceptable de celui-ci et au moins un opioïde pour une utilisation dans la prévention des effets secondaires de cet opioïde, dans lequel les effets secondaires sont la constipation chronique et la sédation, et dans lequel le composé de formule 2-phényl-6-(1H-imidazol-1-yl) quinazoline (CR4056) ou un de ses sels pharmaceutiquement acceptables est administré dans une quantité comprise entre 10 et 400 mg/die.

8. Composition à utiliser selon la revendication 7, dans laquelle l'au moins un opioïde est un agoniste MOR choisi dans le groupe constitué par la morphine, la codéine, le dextrométhorphane, le dextropropoxyphène, le fentanyl, l'hydrocodone, l'hydromorphone, le lévorphanol, la mépéridine, la méthadone, l'oxycodone, l'oxymorphone et/ou leurs sels pharmaceutiquement acceptables.

9. Composition à utiliser selon la revendication 7 ou 8, dans laquelle l'au moins un opioïde est la morphine ou un sel pharmaceutiquement acceptable de celle-ci.

10. Composition à utiliser selon l'une quelconque des revendications 7 à 10, dans laquelle la composition pharmacologique comprend également des excipients pharmaceutiquement acceptables convenant à la forme finale d'administration.

11. Kit pharmaceutique comprenant :
- une ou plusieurs unités discrètes du composé de formule 2-phényl-6-(1Himidazol-1-yl) quinazoline (CR4056) ou un sel pharmaceutiquement acceptable de celui-ci et
- une ou plusieurs unités discrètes d'un opioïde
pour une utilisation simultanée, séquentielle ou séparée dans la prévention des effets secondaires d'au moins un opioïde, les effets secondaires sont choisis parmi la constipation chronique et la sédation.
